(19) Europäisches Patentamt
European Patent Office
Office européen des brevets

(11) Veröffentlichungsnummer: **0 133 504**
**A2**

(12) # EUROPÄISCHE PATENTANMELDUNG

(21) Anmeldenummer: **84108645.7**

(22) Anmeldetag: **21.07.84**

(51) Int. Cl.[4]: **C 07 C 103/64**
C 07 C 102/00, C 07 C 69/54

(30) Priorität: **05.08.83 DE 3328350**

(43) Veröffentlichungstag der Anmeldung:
**27.02.85 Patentblatt 85/9**

(84) Benannte Vertragsstaaten:
**BE DE FR GB NL**

(71) Anmelder: **Röhm GmbH**
**Kirschenallee Postfach 4242**
**D-6100 Darmstadt 1(DE)**

(72) Erfinder: **Arndt, Peter Joseph, Dr.**
**Im Säbchen 9**
**D-6104 Seeheim-Jugenheim(DE)**

(72) Erfinder: **Müller, Manfred**
**Schillerstrasse 55**
**D-6101 Rossdorf 1(DE)**

(72) Erfinder: **Schlosser, Fritz**
**Parkstrasse 48**
**D-6100 Darmstadt(DE)**

(72) Erfinder: **Wenzel, Franz, Dr.**
**Zeyherweg 5**
**D-6100 Darmstadt(DE)**

(54) **Verfahren zur Reinigung von Methacrylamidopropyltrimethylammoniumchlorid und verwandten Verbindungen.**

(57) Zur Reinigung von Methacrylamidopropyltrimethylammoniumchlorid oder ähnlichen quaternierten Aminoalkylestern oder Aminoalkylamiden der Acryl- oder Methacrylsäure von Verunreinigungen, die durch Hofmann'schen Abbau der genannten Verbindungen entstehen können, wie z.B. Allylmethacrylamid, wird eine wäßrige Lösung der Verbindung mit Dichlormethan extrahiert.

EP 0 133 504 A2

Croydon Printing Company Ltd

Verfahren zur Reinigung von Methacrylamidopropyltrimethylammoniumchlorid und verwandten Verbindungen

Die Erfindung betrifft ein Verfahren zur Reinigung von Methacrylamidopropyl-trimethylammoniumchlorid (MAPTAC) und verwandten Verbindungen von mehrfach ungesättigten, bei der Polymerisation vernetzend wirkenden Verunreinigungen.

MAPTAC läßt sich durch Quaternierung von N-Dimethylaminopropylmethacrylamid mit Methylchlorid gewinnen. Für die Ausgangsverbindung N-Dimethylaminopropyl-methacrylamid sind verschiedene Herstellungsverfahren bekannt. Nach DE-PS 2 502 247 erhält man diese Verbindung durch Umsetzung von Methylmethacrylat mit der doppelten Molmenge Dimethylaminopropylamin und thermische Spaltung des dabei entstehenden Michael-Addukts. Auf einfachere Weise entsteht die Verbindung durch Aminolyse von Methylmethacrylat mit Dimethylaminopropylamin in Gegenwart eines Dialkylzinnoxids als Katalysator gemäß DE-PS 2 816 516, vorzugsweise zusätzlich in Gegenwart eines Alkyltitanats gemäß DE-OS 30 48 020.

Die Quaternierungsprodukte von MAPTAC lassen sich in wäßriger

Lösung zu hochmolekularen polykationischen Polymerisaten oder Mischpolymerisaten, die hoch wirksame Sedimentations- und Flockungsmittel sind, polymerisieren. Dabei werden Störungen beobachtet, die allem Anschein nach auf geringe Spuren von vernetzend wirkenden Verunreinigungen zurückzuführen sind. Die Störungen äußern sich darin, daß eine 1 %ige wäßrige Lösung des erhaltenen Polymerisats gallertige, nicht mehr lösbare Rückstände enthält oder daß das Polymerisat in Wasser überhaupt nicht mehr löslich ist. Es wurde festgestellt, daß diese Störungen in um so stärkerem Maße auftreten, je mehr N-Allylmethacrylamid als Verunreinigung in dem MAPTAC enthalten sind. Präparate mit weniger als 10 ppm (Teile pro Million) an N-Allylmethacrylamid erwiesen sich als im wesentlichen frei von den erwähnten Störungen, jedoch waren derartige Präparate nur durch umständliche, in der technischen Praxis kaum anwendbare Verfahren herstellbar.

Die Bildung von N-Allylmethacrylamid aus MAPTAC läßt sich durch Hofmann'schen Abbau gemäß der Reaktionsgleichung

$$CH_2 = \overset{\overset{CH_3}{|}}{C} - CONH - CH_2 - CH_2 - CH_2 - N(CH_3)_3,Cl \longrightarrow$$

$$CH_2 = \overset{\overset{CH_3}{|}}{C} - CONH - CH_2 - CH = CH_2 + N(CH_3)_3 \cdot HCl$$

deuten, ohne daß dieser Bildungsweg als erwiesen gelten kann. N-Allylmethacrylamid wird häufig in Konzentrationen über 10 ppm, z.B. von 40 bis 100 ppm oder sogar darüber, festgestellt. Es ist jedoch keineswegs gesichert, daß N-Allylmethacrylamid die einzige oder wesentlichste Störungsursache ist; vielmehr hat es den Anschein, als ob MAPTAC in der Regel eine Reihe von Verunreinigungen enthielte, die stets in Gesellschaft mit N-Allylmethacrylamid auftreten.

Es war das Ziel der Erfindung, ein technisch leicht durchführbares Verfahren zu finden, nach welchem MAPTAC und verwandte Verbindungen frei von störenden Verunreinigungen, insbesondere mit einem Gehalt von weniger als 10 ppm N-Allylmethacrylamid herstellbar sind. Es wurde gefunden, daß dies durch Extraktion der wäßrigen Lösung mit Dichlormethan möglich ist.

Die Erfindung ist nicht auf MAPTAC beschränkt, sondern ist in entsprechender Weise mit Verbindungen der Formel

$$CH_2 = \overset{R'}{\overset{|}{C}} - CO - X - (CR''_2)_n - CH_2 - CH_2 - N^+R_3,\ A^- \qquad (I)$$

worin R gleiche oder verschiedene Alkylreste, $A^-$ ein Anion einer Säure, R' Wasserstoff oder Methyl, R" Wasserstoff oder einen niederen Alkylrest, X Sauerstoff oder eine NH-Gruppe und n eine ganze Zahl von 0 bis 3 bedeuten, durchführbar. Das gemeinsame Merkmal dieser Verbindungen ist die Fähigkeit, durch Hofmann'schen Abbau in eine zweifach ungesättigte Verbindung der Formel

$$CH_2 = \overset{R'}{\overset{|}{C}} - CO - X - (CR''_2)_n - CH = CH_2 \qquad (II)$$

sowie ein Ammoniumsalz $NR_3 \cdot HA$ überzugehen. Die Polymerisationsfähigkeit der Gruppe $-CH{=}CH_2$ kann die wesentliche Ursache der beobachteten Störungen sein, da sie die Vernetzung des Polymerisats der Verbindung I auslösen kann.

Unter den Verbindungen I sind Abkömmlinge der Methacrylsäure, für die $R'{=}CH_3$ gilt, bevorzugt. Ebenfalls bevorzugt sind Verbindungen mit Amidstruktur, die als X eine NH-Gruppe enthalten. Die Gruppe $CR''_2$ ist in der Regel eine Methylengruppe, jedoch kommen auch andere Alkylidengruppen, wie Äthyliden-, Isopropyliden- oder Isobutylidengruppen, in Betracht. R" enthält

vorzugsweise nicht mehr als 2 C-Atome. Wenn n > 1 ist, können die Gruppen ($CR''_2$) auch ungleich sein. Die Reste R am Stickstoffatom können gleich oder ungleich sein und enthalten vorzugsweise 1 bis 4 C-Atome; bevorzugt sind Methylgruppen. $A^-$ leitet sich vorzugsweise von einer Mineralsäure, ihren Teilestern oder von einer organischen Sulfonsäure ab. Die bevorzugten Säureanionen $A^-$ sind Methosulfat und insbesondere Chlorid, jedoch sind andere Anionen, wie Sulfat (bzw. ein halbes Äquivalent davon), Hydrogensulfat, Bromid, Jodid, Benzolsulfonat, Toluolsulfonat, ebenso geeignet.

Die am meisten bevorzugten Verbindungen der Formel I sind Methacrylamidopropyl-trimethylammoniumchlorid und -methosulfat. Andere bevorzugte Verbindungen sind die entsprechende Acrylamido-Verbindung, sowie Acryl- und Methacryloxyäthyl-trimethylammonium-chlorid oder -methosulfat.

Die Erfindung beruht darauf, daß die mehrfach ungesättigten Verbindungen II und eventuelle weitere begleitende Verunreinigungen in Dichlormethan gut löslich sind, während die salzartigen Verbindungen der Formel I darin völlig unlöslich sind. Andere organische Lösungsmittel, wie z.B. Perchloräthylen, erwiesen sich als ungeeignet zur Extraktion der Verunreinigungen.

Die Konzentration der wäßrigen Lösung der Verbindung I ist nicht wesentlich für das Verfahren der Erfindung, solange keine extrem verdünnten Lösungen eingesetzt werden. Vorzugsweise werden mindestens 20 %ige Lösungen eingesetzt, insbesondere Lösungen bei oder nahe der Sättigungskonzentration bei der Extraktionstemperatur. Für MAPTAC haben sich Konzentrationen von 40 bis

90 Gew.-% bewährt.

Für die Extraktion wird weitgehend reines Dichlormethan bevorzugt, jedoch sind Lösungsmittelgemische aus wenigstens 50 Gew.-% Dichlormethan und zum übrigen Teil aus einem oder mehreren anderen mit Wasser nicht mischbaren Lösungsmitteln, wie anderen Chlorkohlenwasserstoffen oder aromatischen oder aliphatischen Kohlenwasserstoffen, in der Regel verwendbar.

Die Menge des Dichlormethans kann beispielsweise zwischen dem 0,2- und dem 10-fachen des Gewichts der wäßrigen Lösung liegen. Bei diskontinuierlicher Arbeitsweise läßt sich der Reinigungseffekt dadurch verstärken, daß man das Extraktionsmittel in mehrere Portionen zerteilt und die wäßrige Lösung mehrmals mit den einzelnen Portionen extrahiert.

Wie üblich, besteht die Extraktion in einer wenigstens kurzzeitigen innigen Durchmischung der beiden ineinander nicht löslichen Phasen mit anschließender Phasentrennung. Es ist nicht wesentlich, welche der beteiligten Phasen in der anderen Phase zu Tröpfchen zerteilt wird. Zur diskontinuierlichen Verfahrensdurchführung eignen sich Rührgefäße mit einer leistungsfähigen Rührvorrichtung und Bodenventil. Bei kontinuierlicher Arbeitsweise kann eine übliche Flüssigphasen-Extraktionskolonne mit statischen oder dynamischen Mischvorrichtungen und eventuell mit nachgeschaltetem Absitzgefäß verwendet werden. Das Extraktionsmittel kann nach Destillation erneut im Verfahren der Erfindung eingesetzt werden.

Die Extraktion wird in der Regel bei Raumtemperatur, insbesondere im Bereich von 15 bis 30°C durchgeführt, jedoch sind höhere oder tiefere Temperaturen ohne weiteres anwendbar. Eine höhere Extraktionstemperatur kann zweckmäßig sein, um bei einer höheren Sättigungskonzentration der wäßrigen Lösung arbeiten zu können, jedoch sind Temperaturen oberhalb der Siedetemperatur des Extraktionsmittels wegen der dann erforderlichen Druckapparaturen nicht zweckmäßig.

In der Regel liegt der Gehalt an Verbindungen der Formel II in der wäßrigen Lösung nach einem, höchstens nach drei Extraktionsschritten unter 10 ppm. Die so gereinigte Lösung kann unmittelbar zur Herstellung unvernetzter, extrem hochmolekularer Homo- oder Mischpolymerisate der Verbindung I in Form einer wäßrigen Polymerisatlösung oder eines löslichen Gels eingesetzt werden. Gewünschtenfalls kann die Lösung aber auch zur Trockne eingedampft werden, um die Verbindung I in reiner Form zu gewinnen. Dabei ist schonend zu verfahren, um nicht erneut durch Hofmann'schen Abbau die Bildung von Verunreinigungen zu veranlassen.

Beispiel

1000 g einer 50 %igen wäßrigen Lösung von MAPTAC, die (nach HPLC-Bestimmung) 45 ppm N-Allylmethacrylamid enthält, wird

a) zweimal mit je 500 ml Dichlormethan
oder b) dreimal mit je 200 ml Dichlormethan

in einem Schütteltrichter intensiv durchmischt und nach dem Absetzen des Dichlormethans abgetrennt. In der dritten Extraktionsmittelmenge sind nur Spuren von N-Allylmethacrylat im Bereich unter 0,1 ppm nachweisbar.

In der gereinigten Lösung sind in beiden Fällen danach weniger als 1 ppm N-Allylmethacrylamid nachweisbar. Nach Polymerisation werden hochmolekulare Produkte erhalten, die 1 %ig in Wasser homogene Lösungen von 6000 bis 10.000 mPas ergeben.

Vergleichsversuch

Durch viermalige Extraktion mit je 500 ml Perchloräthylen bleibt der Gehalt an N-Allylmethacrylamid in der eingesetzten MAPTAC-Lösung praktisch unverändert.

Daraus hergestellte Polymerisate sind in Wasser unlöslich, also vollständig vernetzt.

Verfahren zur Reinigung von Methacrylamidopropyltrimethylammoniumchlorid und verwandten Verbindungen

Patentansprüche

1. Verfahren zur Reinigung einer Verbindung der Formel

$$CH_2=\overset{R'}{\overset{|}{C}} - CO - X - (CR''_2)_n - CH_2 - CH_2 - N^+R_3,A^- \qquad (I)$$

von Verunreinigungen enthaltend eine Verbindung der Formel

$$CH_2 = \overset{R'}{\overset{|}{C}} - CO - X - (CR''_2)_n - CH = CH_2,$$

worin jeweils R gleiche oder verschiedene Alkylreste,

R' Wasserstoff oder Methyl,
R'' Wasserstoff oder einen Niederalkylrest,
X Sauerstoff oder die Gruppe -NH-
$A^-$ ein Anionäquivalent einer Säure
n eine ganze Zahl von 0 bis 3 bedeuten,

dadurch gekennzeichnet,

daß man eine wäßrige Lösung der Verbindung der Formel I mit Dichlormethan extrahiert.

2. Verfahren nach Anspruch 1, dadurch gekennzeichnet, daß als Verbindung der Formel I Methacrylamidopropyltrimethylammoniumchlorid eingesetzt wird, welches als Verunreinigung N-Allylmethacrylamid enthält.